# EUROPEAN PATENT SPECIFICATION

(11) **EP 2 396 658 B1**
(45) Date of publication and mention of the grant of the patent: **16.12.2015**
(21) Application number: 10704845.6
(22) Date of filing: 16.02.2010
(51) Int. Cl.: G01N 33/574

(54) **ASSAY FOR DETECTION OF ADRENAL TUMOUR**
ASSAY ZUR DETEKTION VON NEBENNIERENTUMOR
DOSAGE POUR LA DETECTION DE TUMEUR SURRENALE

(30) Priority: 16.02.2009 GB 0902565
(43) Date of publication of application: 21.12.2011
(73) Proprietor: The University of Birmingham, Edgbaston Birmingham West Midlands B15 2TT (GB)
(72) Inventor: ARLT, Wiebke, Selly Park Birmingham B29 7EX (GB); STEWART, Paul, Michael, Earlswood Solihull B94 6BY (GB)
(74) Representative: Elsy, David
(86) International application number: PCT/GB2010/000274
(87) International publication number: WO 2010/092363

(56) References cited:
- SHACKLETON ET AL: "Mass spectrometry in the diagnosis of steroid-related disorders and in hypertension research" JOURNAL OF STEROID BIOCHEMISTRY AND MOLECULAR BIOLOGY, ELSEVIER SCIENCE LTD., OXFORD, GB LNKD- DOI:10.1016/0960-0760(93)90132-G, vol. 45, no. 1-3, 1 April 1993 (1993-04-01), pages 127-140, XP023549406 ISSN: 0960-0760 [retrieved on 1993-04-01]
- MASER-GLUTH C ET AL: "Metabolism of glucocorticoids and mineralocorticoids in patients with adrenal incidentalomas" EUROPEAN JOURNAL OF CLINICAL INVESTIGATION, vol. 30, no. Suppl. 3, December 2000 (2000-12), pages 83-86, XP002578258 ISSN: 0014-2972
- CAULFIELD MICHAEL P ET AL: "The diagnosis of congenital adrenal hyperplasia in the newborn by gas chromatography/mass spectrometry analysis of random urine specimens" JOURNAL OF CLINICAL ENDOCRINOLOGY AND METABOLISM, vol. 87, no. 8, August 2002 (2002-08), pages 3682-3690, XP002578259 ISSN: 0021-972X
- M. Biehl, B. Hammer, P. Schneider: "Matrix Learning in Learning Vector Quantization"[Online] 1 December 2006 (2006-12-01), XP002578260 Retrieved from the Internet: URL:http://www.in.tu-clausthal.de/fileadmi n/homes/techreports/ifi0614biehl.pdf> [retrieved on 2010-04-16]

## Description

The invention relates to a method for detecting or monitoring an adrenal tumour by detecting one or more adrenal steroid metabolites. Assay kits and computer systems relating to such methods are also provided. The invention is amenable to automation and allows discrimination between malignant and benign tumours.

The adrenal glands, which are also known as suprarenal glands, are star-shaped endocrine glands that sit on top of the kidneys. They are mainly responsible for regulating the stress response through the synthesis of corticosteroids and catecholamines, including cortisol and adrenaline.

Corticosteroids secreted from the adrenal gland are essential for life. Adrenal insufficiency arises either through deficiency of the gland itself (Addison's disease, congenital adrenal hyperplasia) or through impaired secretion of trophic hormones such as ACTH from hypothalamic/ pituitary disorders.

Paradoxically overproduction of steroids from the adrenal gland also leads to significant human disease. Cortisol excess results in Cushing's syndrome characterised by obesity, diabetes, hypertension, infections and increased cardiovascular mortality. Aldosterone excess or Conn's syndrome is a common cause of mineralocorticoid hypertension that is now known to explain 10% of all cases of hypertension (i.e. 1% of the population). Rarely, autonomous adrenal androgen excess can cause virilisation in women (clitoromegaly, hirsutism). Most of these cases arise because of autonomous but benign adenomas within the adrenal cortex but as a group they present a diagnostic challenge. This is compounded by the fact that up to 5% of the adult population harbour so-called "adrenal incidentalomas", i.e. adrenal tumours that are discovered incidentally upon imaging arranged for other reasons. With the proliferation of diagnostic imaging this condition is placing an increased burden on the NHS and patients alike. Most incidentalomas are non-functioning but 15% are associated with mild glucocorticoid excess resulting in diseases such as obesity, osteoporosis and diabetes. However, secretion occurs at a lower level than the classical Cushing's and Conn's adenomas referred to above and conventional cut-offs of serum hormone assessments are often not specific and sensitive enough for a diagnosis. In addition to the exclusion of tumour-related hormone excess, adrenal incidentalomas require assessment of their malignant potential. Malignancy risk increases with size of the tumour and some imaging features are suggestive of malignancy, but diagnostic tools for sensitive and specific detection of malignancy, i.e. differentiating a benign adrenal adrenoma from adrenal carcinoma in this cohort are lacking.

Adrenal carcinoma is a rare tumour with an annual incidence of 1/million. Their aetiology is unknown but tumours are invasive and aggressive and treatment options poor such that the 5-year survival from diagnosis is less than 35%. Early diagnosis followed by complete surgical excision represents the only hope of cure. Post surgery we currently lack the ability to monitor disease burden and recurrence in affected cases. Many of these tumours are functional and secrete glucocorticoids, androgens, mineralocorticoids or steroid precursors, but in a significant percentage of cases serum hormone measurements fail to pick up abnormalities.

Urinary steroid profiles now represent a unique source of hope for management of these conditions, both in terms of diagnosis and monitoring. In the age of "omics" technologies imperfect quantification of a few diagnostic analytes diagnostic of endocrine conditions is no longer sufficient. Immunoassay, which has been the backbone of endocrine investigation for decades, no longer has the accuracy demanded for comprehensive clinical investigation or basic hormonal studies. For many years the measurement of a few secreted hormones, e.g. cortisol or aldosterone, was considered to be sufficient for diagnosing steroid disorders, but increasingly it has been shown that steroid metabolism in addition to biosynthesis is a major concept. This is exemplified in conditions such as Apparent Mineralocorticoid Excess and apparent cortisone reductase deficiency. Current clinical tests cannot achieve this level of detailed investigation. The term metabolomics has been introduced to emphasize the new requirement for a more global approach to biochemical analysis. While a metabolome includes all biologically produced components, a subdivision termed a "metabolic profile" is generally more useful within the clinical context. A metabolic profile is defined as the analysis of a group of metabolites either related to a specific metabolic pathway or a class of compounds. While steroid metabolic profiles have been around for many years the Inventor's recent work has suggested a broader role for diagnosing and monitoring human disease.

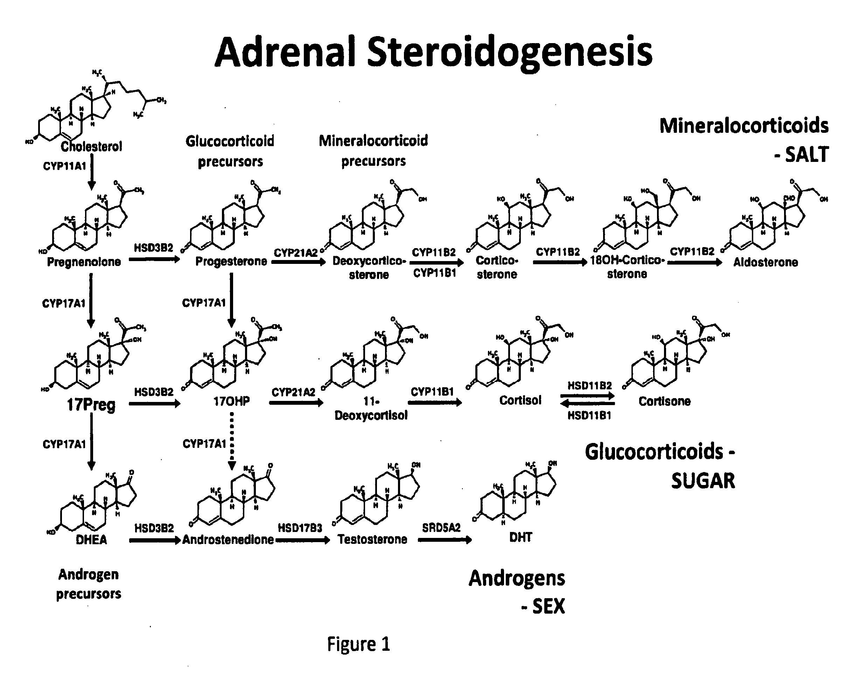

Figure 1 shows adrenal steriodogenesis and indicates the range of different compounds produced by the adrenal gland and which are subsequently metabolised or utilised in the body.

The use of gas chromatography/mass spectrometry (GC/MS) and the analysis of over 50 separate adrenal steroids/metabolites have demonstrated that they can accurately profile glucocorticoid, minerelacorticoid and sex steroid pathways and absolute values of secretion.

The Inventors have studied such steroids and metabolites in urine samples from healthy individuals and individuals with benign adrenocortiocal adenomas (ACA) and adrenocortical carcinomas (ACC), respectively.

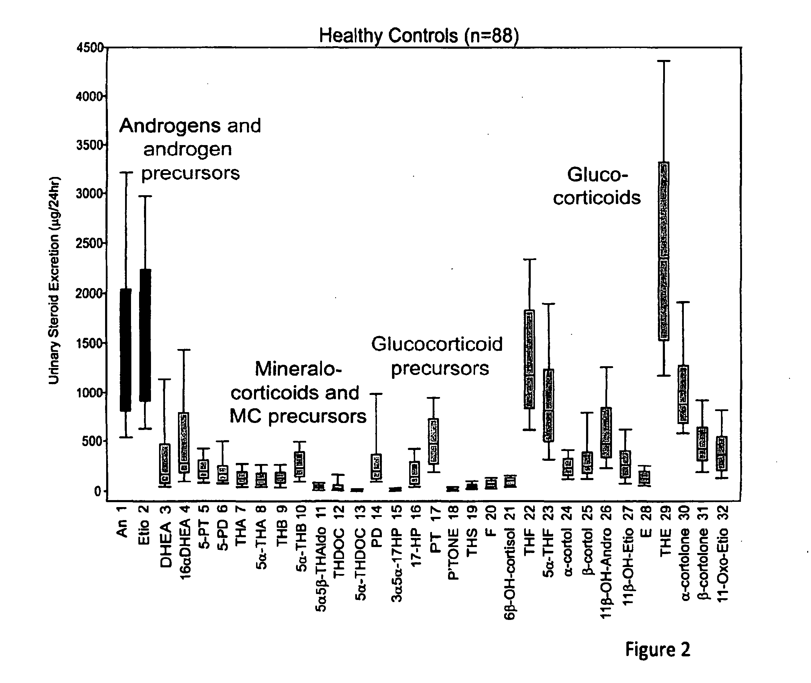

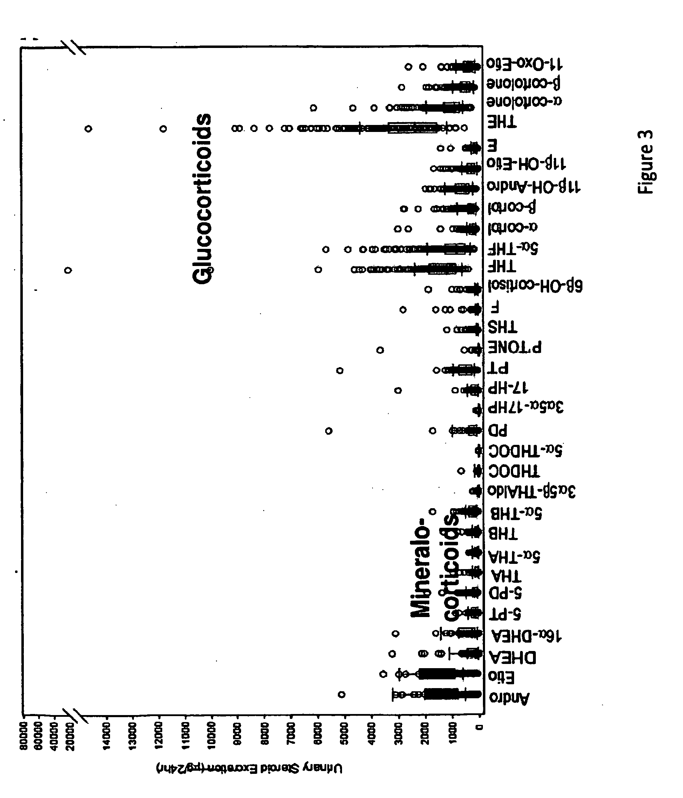

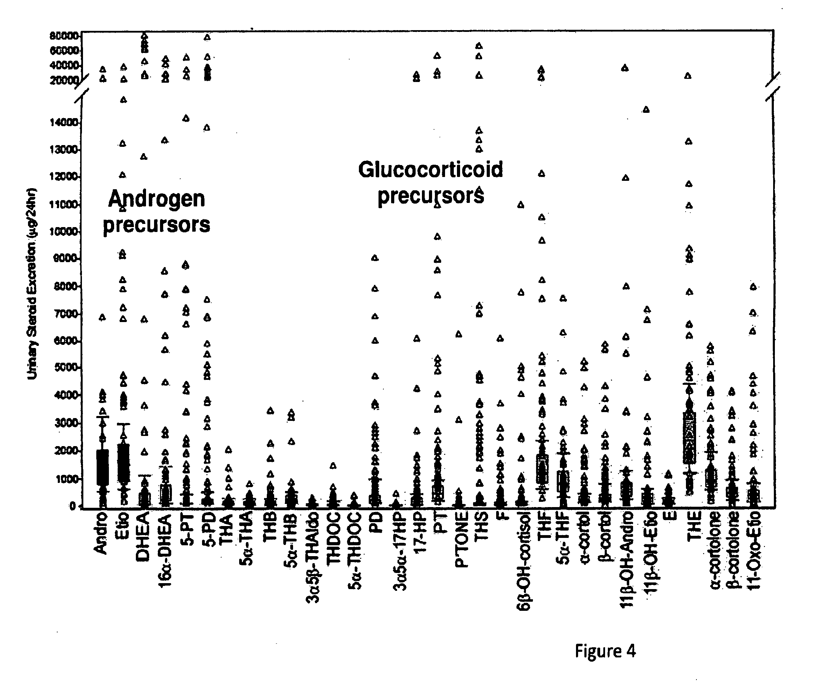

Figures 2, 3 and 4 show the range of the amounts of steroid excreted per 24 hours, from a range of patients, including healthy controls, those with adrenocortical adenomas and those with adrenocortical carcinomas as detected by GC/MS.

The urinary steroid metabolites that are quantified by a single GC/MS scanning run, together with the serum steroids they derive from, and commonly used abbreviations for the urinary metabolites are listed in Table 1 below.

**Table 1**

| Steroids and steroid metabolites analyzed in a single diagnostic run by gas chromatography/mass spectrometry (GC/MS). | | | | |
|---|---|---|---|---|
| **No.** | **Abbreviation** | **Common name** | **Chemical name** | **Metabolite of** |
| **Androgen metabolites** | | | | |
| 1 | An | Androsterone | 5α-androstan-3a-ol-17-one | Androstenedione, testosterone, 5a-dihydrotestosterone |
| 2 | Etio | Etiocholanolone | 5β-androstan-3a-ol-17-one | Androstenedione, testosterone |

| **Androgen precursor metabolites** | | | | |
|---|---|---|---|---|
| 3 | DHEA | Dehydroepiandrosterone | 5-androsten-3β-ol-17-one | DHEA + DHEA sulfate (DHEAS) |
| 4 | 16α-OH- DHEA | 16α-hydroxy-DHEA | 5-androstene-3β,16α-diol-17-one | DHEA + DHEAS |
| 5 | 5-PT | | 5-pregnene-3β,17, 20α-triol | |
| 6 | 5-PD | | 5-pregnene-3β, 20α-diol and 5, 17, (20)-pregnadien-3β-ol | Pregnenolone |

| **Mineralocorticoid metabolites** | | | | |
|---|---|---|---|---|
| 7 | THA | Tetrahydro-11- dehydro-corticosterone | 5β-pregnane-3α,21-diol,11, 20-dione | Corticosterone, 11-dehydro-corticosterone |
| 8 | 5α-THA | 5α-tetrahydro-11-dehydro-corticosterone | 5α-pregnane-3α, 21-diol-11,20-dione | Corticosterone, 11-dehydrocorticosterone |
| 9 | THB | Tetrahydro-corticosterone | 5β-pregnane-3α, 11β, 21-triol-20-one | Corticosterone |
| 10 | 5α-THB | 5α-tetrahydro-corticosterone | 5α-pregnane-3α, 11β, 21-triol-20-one | Corticosterone |
| 11 | 3α5β-THALDO | Tetrahydro-aldosterone | 5β-pregnane-3α, 11β, 21-triol-20-one-18-al | Aldosterone |

| **Mineralocorticoid precursor metabolites** | | | | |
|---|---|---|---|---|
| 12 | THDOC | Tetrahydro-11-deoxycorticosterone | 5β-pregnane-3α, 21-diol-20-one | 11-deoxycorticosterone |
| 13 | 5α-THDOC | 5α-tetrahydro-11-deoxycorticosterone | 5α-pregnane-3α, 21-diol-20-one | 11-deoxycorticosterone |

| **Glucocorticoid precursor metabolites** | | | | |
|---|---|---|---|---|
| 14 | PD | Pregnanediol | 5β-pregnane-3α, 20a-diol | Progesterone |
| 15 | 3α5α-17HP | 3α, 5α-17-hydroxy-pregnanolone | 5α-pregnane-3α, 17α-diol-20-one | 17-hydroxy-progesterone |
| 16 | 17HP | 17-hydroxy-pregnanolone | 5β-pregnane-3α, 17α,-diol-20-one | 17-hydroxy-progesterone |
| 17 | PT | Pregnanetriol | 5β-pregnane-3α, 17α, 20α-triol | 17-hydroxy-progesterone |
| 18 | PTONE | Pregnanetriolone | 5β-pregnane-3α, 17, 20α-triol-11-one | 21-deoxycortisol |
| 19 | THS | Tetrahydro-11-deoxycortisol | 5β-pregnane-3α, 17, 21-triol-20-one | 11-deoxycortisol |

| **Glucocorticoid metabolites** | | | | |
|---|---|---|---|---|
| 20 | F | Cortisol | 4-pregnene-11β, 17, 21-triol-3, 20-dione | Cortisol |
| 21 | 6β-OH-F | 6β-hydroxy-cortisol | 4-pregnene-6β, 11β, 17, 21-tetrol-3, 20-dione | Cortisol |
| 22 | THF | Tetrahydrocortisol | 5β-pregnane-3α, 11β, 17, 21-tetrol-20-one | Cortisol |
| 23 | 5α-THF | 5α-tetrahydrocortisol | 5α-pregnane-3α, 11β, 17, 21-tetrol-20-one | Cortisol |
| 24 | α-cortol | α-cortol | 5α-pregnan-3α, 11β, 17, 20β, 21-pentol | Cortisol |
| 25 | β-cortol | β-cortol | 5β-pregnan-3α, 11β, 17, 20β, 21-pentol | Cortisol |
| 26 | 11b-OH-An | 11β-hydroxy-androsterone | 5α-androstane-3α, 11β-diol-17-one | Cortisol (+ Androgens) |
| 27 | 11b-OH-Et | 11b-hydroxy-etiocholanolone | 5β-androstane-3α, 11β-diol-17-one | Cortisol (+ Androgens) |
| 28 | E | Cortisone | 4-pregnene-17α, 21-diol-3, 11, 20-trione | Cortisol |
| 29 | THE | Tetrahydrocortisone | 5β-pregnene-3α, 17, 21-triol-11, 20-dione | Cortisol |
| 30 | α-cortolone | α-cortolone | 5β-pregnane-3α, 17, 20α, 21-tetrol-11-one | Cortisol |
| 31 | β-cortolone | β-cortolone | 5β-pregnane-3α, 17, 20β, 21-tetrol-11-one | Cortisol |
| 32 | 11-oxo-Et | 11-oxo-etiocholanolone | 5β-androstan-3α-ol-11, 17-dione | Cortisol (+ Androgens) |

The profiling shown in the attached figures indicates that the profiles obtained are complex.

The Inventors therefore utilised bio computational data analysis to identify those metabolites having a significant difference in their production between patients with ACA (benign tumours) and patients with ACC (malignant tumours).

The technique utilised is Matrix Relevance Learning Vector Quantization (MRLVQ). MRLVQ is a significant conceptual extension of standard Learning Vector Quantisation which is reviewed in, for example, Kohonen T, self-organising maps, Springer (1997).

LVQ methods serve for the classification of data that can be represented as *N*-dimensional vectors. In the application at hand, the components or *features* are, for instance, *N =* 32 steroid concentrations measured per patient.

This resulted in the identification of a group of a number of metabolites which are able to be used to detect and monitor tumours. They allow the differentiation between, for example, benign and malignant tumours and the progression of treatments to be readily monitored. The ability to test samples rapidly, for example, using a urine sample, has considerable advantages because of its simplicity. Moreover, it is possible to collect urine over a period of time, for examples 24 hours, which allows the amount of the metabolite produced in urine over a set time period, to be utilised. Using samples of blood or serum, only allows the concentration of a sample to be determined at a set time. Moreover, the collection of blood and serum is more invasive and more complex.

The invention provides a method of detecting or monitoring an adrenal tumour, comprising measuring the concentration or amount of:
THS (tetrahydro-11-desoxycortisol), 5-PT (5-pregnenetriol), 5-PD (pregnenediol), PD (pregnanediol), PT (pregnanetriol), Et (etiocholanolone), TH-DOC (tetrahydro-11-deoxycorticosterone), 5α-THA (5α-tetra-11-hydrocorticosterone) and 5α-THF (5α-tetrahydrocortisol).

Additionally An (androsterone) may be measured.

The absolute concentration of compounds within a known volume of the sample of urine may be used to determine the amount of compound produced by a patient within a time period, for example, in urines collected from a patient in 6, 12, 18 or 24 hours. This allows a more accurate representation of the amount of compound to be identified and allows for variations due to, for example, the dilution of urine as a result of the patient drinking more or less fluids at different times of the day.

Moreover, the Applicant has unexpectedly demonstrated that this technique is relatively easily and rapidly carried out by, for example LC/MS/MS, reducing cost and reducing the time to obtain the necessary date. This produces a high throughput assay amenable to automation.

The compound measured is preferably THS. 1, 2, 3, 4, 5, 6, 7, 8, 9 or 10 of the compounds are preferably measured. Most preferably 3, 4, more preferably 5 of the compounds are measured.

The method preferably comprises measuring THS, 5-PT and 5-PD. THS, 5-PT, 5-PD, PD and PT may be measured. THS, 5-PT, 5-PD, PD, PT, Et, TH-DOC, 5α-THA and 5α-THF may be measured. All of the compounds may also be measured.

Preferably, the ratio of androsterone : etiocholanolone (An : Et) is also determined. This is because the ratio of androsterone : etiocholanolone has been found to be elevated in malignant tumours.

The above compounds are metabolites of precursors of the final compounds found in normal adrenal glands. It is believed the increased level of precursors may be due to the different differentiation of cells within, for example, malignant, versus benign tumours.

The method of the invention may also comprise measuring the concentration amount of 6 B hydroxycortisol (6 ß-OH-Cortisol).

6 ß-OH-Cortisol is generated in huge amounts during mitotane treatment. Mitotane is a medication used in the treatment of adrenocortical carcinomas. It was originally produced by Bristol Myers Squibb SpA. Usually the metabolite (6 ß-OH-Cortisol) is relatively low in urine. However, mitotane induces the enzyme responsible for 6 ß-hydroxylation, which leads to the increase in the metabolite in urine.

Accordingly, 6 ß-OH-Cortisol may be used to monitor mitotane therapy.

The tumour may be ACA or ACC. The method preferably allows discrimination between benign and malignant tumours.

The sample is preferably from a mammal, especially a human. The sample may be from a human adult, for example one 16 years old.

A method of monitoring the treatment of an adrenal tumour is also described, comprising providing a sample of urine from a patient that has been treated with mitotane and determining the concentration or amount of 6 ß-OH-Cortisol in the sample. This may be compared to, for example, a normal level identified in patients who have either not been treated with mitotane or who have been treated with a standardised dose of mitotane, in order to establish whether the mitotane is being metabolised correctly. Alternatively, it may be used to compare a sample take before or earlier in the mitotane treatment.

Preferably the adrenal tumour detected or monitored is adrenocortical adenoma or an adrenocortical carcinoma.

A method detecting or monitoring an adrenal tumour is also described, comprising measuring the concentration in a tissue, such as blood or serum, of one or more compounds selected from 11-desoxycortisol, 17-hydroxy-pregnenolone, pregnenolone, progesterone and 17-hydroxy progesterone.

Such compounds are the precursors of the metabolites identified in urine. Hence, it is expected that such compounds may be detected in, for example, blood or serum. Preferably 1, 2, 3, 4 or 5 of the compounds are determined, especially 3, 4 or 5. Preferably the method comprises measuring 11-desoxycortisol sterol. The method may comprise measuring 11-desoxycortisol sterol, 17-hydroxy-pregnenolone and pregnenolone.

Testosterone may additionally be measured.

The concentration of the or each compound is compared to a predetermined value. For example, a predetermined value may be a reference value associated with samples from non-tumourous patients, patients with benign tumour and/or samples from patients with malignant tumour. This may be scored to give an indication of whether, for example, the adrenal tumour is benign or malignant. The amount of an individual compound has been noted to vary from patient to patient. Hence, preferably 3, 4, 5 or more of the compounds utilised in any aspect of the invention, are measured and are compared to the reference value for each compound. A score is then determined for each compound against the reference value. This provides a more accurate prediction since, if one of the compounds is produced in a lower concentration, then this is often counteracted by increased concentrations for other compounds, thus leading to more accurate data and prediction. The score may, for example, be based on the percentage amount above or below the reference value for the compound.

The methods of the invention also allow the samples to be utilised to follow the treatment of a patient. For example, a sample of urine, blood or serum may be taken from a patient prior to either surgical removal of the tumour or prior to treatment of the tumour, with an anti-tumour agent. A sample is then taken after the treatment and compared. Alternatively, a number of samples may be taken over a period of time to follow the progression of the treatment, or success of the treatment.

The methods of the invention may also be used for identifying or monitoring for treatment of an adrenal tumour, by comparing the concentration of a compound as defined above, with a concentration of the or each compound from one or more samples obtained from a patient not treated with the drug.

The concentration or amount of the or each compound in the sample may be determined by methods generally known in the art, including, for example, gas chromatography/mass spectrometry or liquid chromatography/tandem mass spectrometry (GC/MS or LC/MS/MS).

Typically the LC/MS is a uPLC tandem mass spectrometer. This may be used in positive ion mode.

GC/MS and LC/MS are especially preferred as these allow the rapid detection of samples and can be readily automated.

GC/MS and LC/MS are especially preferred as these allow the rapid detection of samples and can be readily automated.

As indicated above, the concentrations obtained may be compared to reference (or normalised) values obtained from samples of, for example, urine or serum, obtained from non-tumourous, benign or malignant tumour containing patients.

The invention therefore provides a computer system having a processor, a memory and an input for receiving data obtained from a method of the invention and as defined in claim 10.

Preferably the data is compared to the reference value for each compound measured to produce a ranking value indicative of the presence of the tumour and/or whether the tumour is benign or malignant. Hence, for example, if 5 compounds are measured, then each is compared to a reference value and is given a value or number in comparison to that reference value. Typically from the set of these numbers a ranking value is computed by MRLVG to give an indication of whether the tumour is benign or malignant.

Steroid concentrations may be expressed on a logarithmic scale.

Computer programs comprising instructions which, when run on a computer with a processor, memory and input, configure it to the computer according to the invention are also provided.

Kits for measuring THS, 5-PT and 5-PD, and optionally PD, PT, TH-DOC, 5α-THA, 5α-THF and Et in the samples, for example urine or serum, are also provided. Such kits may contain, for example, standard concentrations of the compounds, such as THS, 5-PT, 5-PD, and optionally PD and/or PT. The kit may detect THS, 5PT, 5PD, PD, PT, Et, TH-DOC, 5α-THA and 5α-THF. The kit may also contain one or more solvents, or other agents for use with the method used to detect the compounds, instructions, solvents, antibodies or detection agents.

The kit may also comprise software to allow the concentration or amount obtained from the method to be compared to the reference values obtained from known samples or tumours.

The reference values obtained from known samples from patients without tumours, or with benign or malignant tumours, may be stored on a separate database.

The concentration or amount of the compounds in a patient's sample of, for example urine or serum, may be obtained remotely from the reference values, and the data sent, for example via an internet access, to a computer storing the reference values. The reference values may then be compared with the concentration or amount obtained, to produce the scope or value and an indication of, for example, whether the patient has a tumour and/or whether the tumour is benign or malignant.

The kit may be adapted for use with, for example, GC/MS or, for example, LC/MS/MS to allow for high throughput analysis.

The invention also provides an adrenal tumour detection or monitoring apparatus comprising a computer system according to the invention and/or a computer program according to the invention, in combination with a kit according to the invention.

Primary hyperaldosteronism (PA) has been found to be a common cause of "essential" or "mineralocorticoid" hypertension (Stewart P.M. and Allolio B., Clin. Endocrinol. (2010) 72, 146-148). PA may be due to a solitary autonomous secreting adenoma, bilateral idiopathic hyperplasia and rarely familial causes. The data set used to produce the assay and kits described above, also identified that unilateral adrenocortical adenomas (ACA), a major cause of PA, produce high levels of mineralcorticoid compounds, compared to bilateral hyperplasia. Conventional sampling techniques by adrenal vein sampling are difficult, so a better way of testing for ACA is required.

A method of differentiating between bilateral adrenal hyperplasia and unilateral adrenocortical adenoma in causing mineralocorticoid excess is also described, comprising measuring the concentration or amount of one or more compounds in urine selected from:
THA, 5α-THA, THB, 5α-THB, 3α 5βTHAldo and 5α-TH-DOC.

Preferably 1, 2, 3, 4, 5, 6 or 7 compounds are assayed.

Differences in mineralocorticoid patterns will help to differentiate between a unilateral adrenocortical adenoma and bilateral hyperplasia.

Kits for detecting one or more of the minerallocorticoid compounds are also described. Preferably the kit detects 1, 2, 3, 4, 5, 6 or 7 of the compounds. The kit may be for GC/MS or LC/MS.

The invention will now be described by way of example only, with reference to the following figures:
Figure 1 shows Adrenal Steroidgenesis.
Figure 2 shows Urinary Steroid Excretion in micrograms per 24 hours for healthy control patients (for abbreviations, see Table 1).
Figure 3 shows GC/MS Steroid Profiling in Adrenocortical Adenomas (ACA; n = 104).
Figure 4 shows GC/MS Steroid Profiling in Adrenocortical Carcinomas (ACC; n = 45).
Figure 5 shows visualisation of ACAx and ACC* data clusters obtained by MRLVQ, with ACA clustered predominantly to the right of ACC.
Figures 6 shows a typical diagonal elements of relevance matrix obtained by MRLVQ (a).
   The most relevant markers have been placed in order of importance (b). The numbers correlate to the compounds listed in Figure 2. Higher values indicate a significant role of the corresponding steroid in the classification.
Figure 7 shows MRLVQ classification of markers according to the invention (a) ACA, (b) ACC, (c) diagonal relevancy for An, Et, 5-PT, 5-PD, THS.
Figure 8 a shows the selectivity of the preferred markers of the invention in (left to right) control, ACA and ACC samples.
Figure 8b shows the number of steroids found to be increased out of the 9 selected markers identified as most differentiating in patients with ACC (n = 45), patients with ACA (n=102) in comparison to healthy controls (n=88) (left to right controls, ACA, ACC).
Figure 8c shows the type of increase selective markers for ACC (right) Vs ACA (left) for over two fold above the reference range.
Figure 9 shows amount of markers in patients in ACA compared to ACC.
Figure 10 shows a receiver operated characteristic curve of assay sensitivity against 1-specificity for 3, 6 and 9 of the compounds of the invention (lower curves) against using 32 steroids as markers. The markers were (3)THS, 5-PTm 5-PD, (6) THS, 5-PD, 5-PT, TH-DOC, 5α-THA, PT and (9) THS, 5-PD, 5-PT, TH-DOC, 5α-THA, PT, 5α-THF, PD, Et (Etio).
Figure 11 shows that total steroid excretion is tumour size independent.
Figure 12 shows minerallocorticoid levels in ACA and adrenal tumours.

### Materials and Methods

Urine samples were obtained from patients without tumours, patients with benign adrenocortical adenomas (ACA) and patients with adrenocortical carcinomas (ACC) respectively.

Urinary steroid metabolite excretion was performed by quantitative GC/MS selected-ion monitoring (SIM) analysis, employing the method by Shackleton (References 1-3). In brief, analysis of steroids is carried out after enzymatic release from conjugation and extraction, followed by GC/MS quantification.
1 Shackleton CH. Mass spectrometry in the diagnosis of steroid-related disorders and in hypertension research. J Steroid Biochem Mol Biol 1993; 45: 127-40.
2 Caulfield MP, Lynn T, Gottschalk ME, et al. The diagnosis of congenital adrenal hyperplasia in the newborn by gas chromatography/mass spectrometry analysis of random urine specimens. J Clin Endocrinol Metab 2002; 87: 3682-90.
3 Arlt W, Walker EA, Draper N, Ivison HE, Ride JP, Hammer F, Chalder SM, Borucka-Mankiewicz M, Hauffa BP, Malunowicz EM, Stewart PM, Shackleton CH. Congenital adrenal hyperplasia caused by mutant P450 oxidoreductase and human androgen synthesis: analytical study. Lancet. 2004 Jun 26;363(9427):2128-35.

The amounts of steroid excreted per 24 hours, is shown in Figures 2, 3 and 4. The abbreviations are listed in Table 1, above.

The data obtained was analysed using MRLVQ P. Scheider, M. Biehle, B. Hanner, Neural Computation (2009) 3532-3561.

As with all machine learning techniques, LVQ depends on available example data; in this case: vectors of steroid concentrations labelled by the expert physicians as "ACC" or "ACA". "ACA" means tumours assessed as Adrenocortical Adenomas and ACC means Adrenocortical Carcinomas. In a *training phase,* LVQ determines from these examples a set of typical vectors, so-called prototypes, representing the different classes. It is possible to represent each class by a number of prototypes.

After training, any new feature vector (i.e. patient) can be classified by the system. To this end, its distance from each of the prototypes is determined. Usually, the new data is assigned to the class which is represented by the closest prototype. In original LVQ, this distance measure is fixed and predefined, e.g. according to human insight, the by far the most frequently used being the Euclidean distance in *N*-dimensional space.

In Relevance Learning, one adaptive weighting factor is assigned to each of the N features. This principle has been developed further to utilise a full matrix of relevances in MRLVQ. Correlations between different features can be taken into account. Further discussions of the techniques utilised are shown in the following documents:
M. Biehl, B. Hammer, P. Schneider Matrix Learning in Learning Vector Quantization Technical Report, Ifl Technical Report Series Ifl-06-14, TU Claushal, 2006
P. Schneider, M. Biehl, B. Hammer, Relevance Matrices in Learning Vector Quantization In: Proc. Of the 15th European Symposium on Artificial Neural Networks ESANN 2007, M. Verleysen (ed.), d-side publishing, pp. 37-42, 2007

### Results

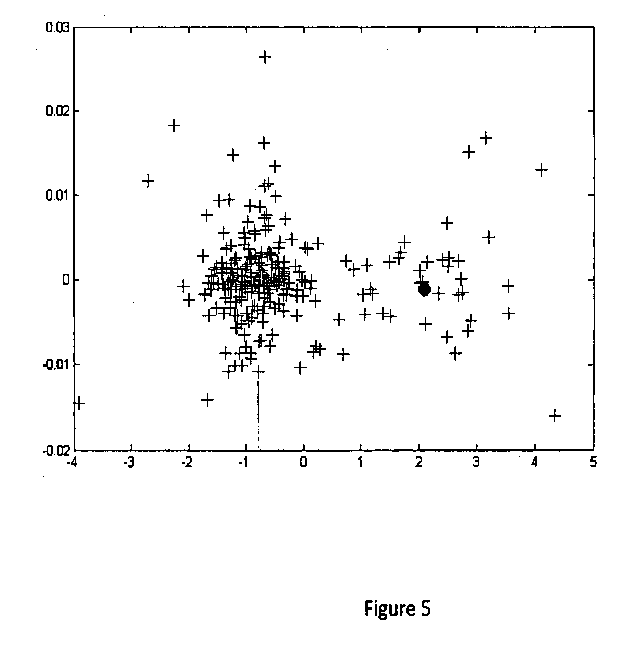

MRLVQ allowed the visualisation of clusters of data (see for example Figure 5).

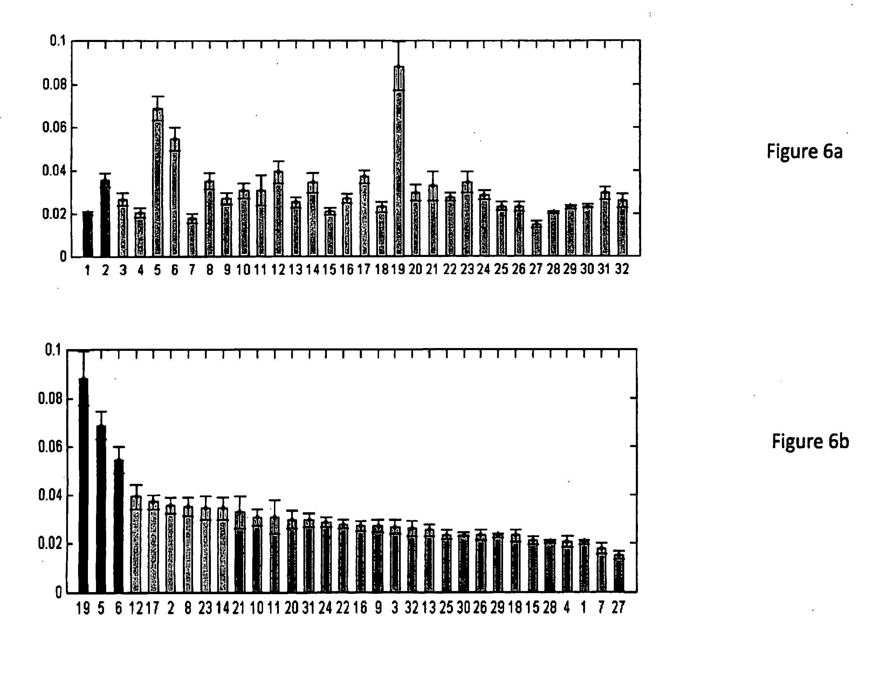

Figure 6 shows a typical relevance matrix print out for the compounds tested, with the compounds of the invention highlighted.

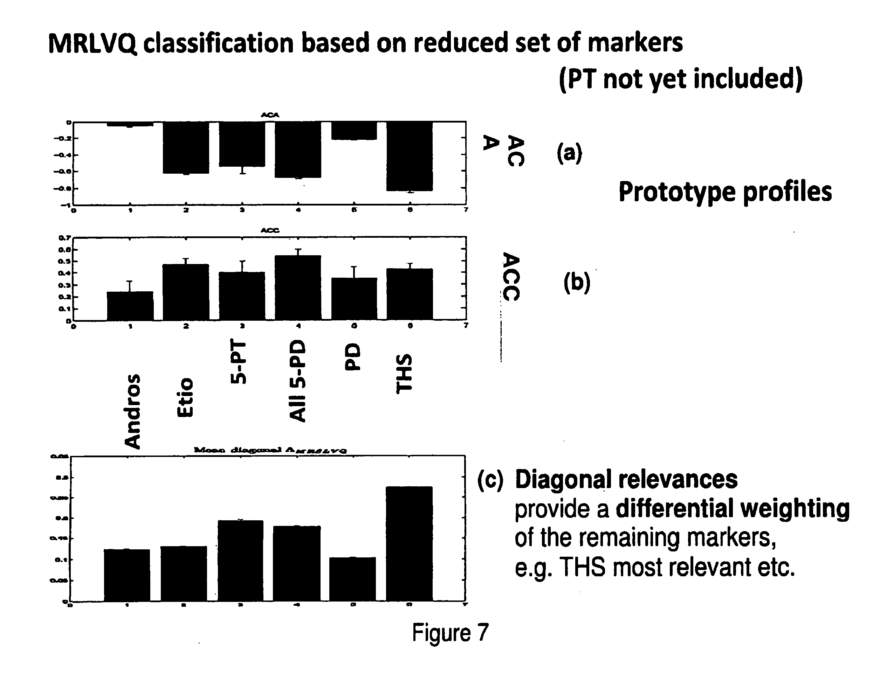

The MRLVQ initially identified 7 compounds An, Et, 5PT, PD, 5PD, THS and PT, as of particular relevance to identifying ACA and ACC. Figure 7 shows, for example, prototype profiles for 6 of those compounds. The technique allows differential weighting to be put on each marker, showing THS to be most relevant.

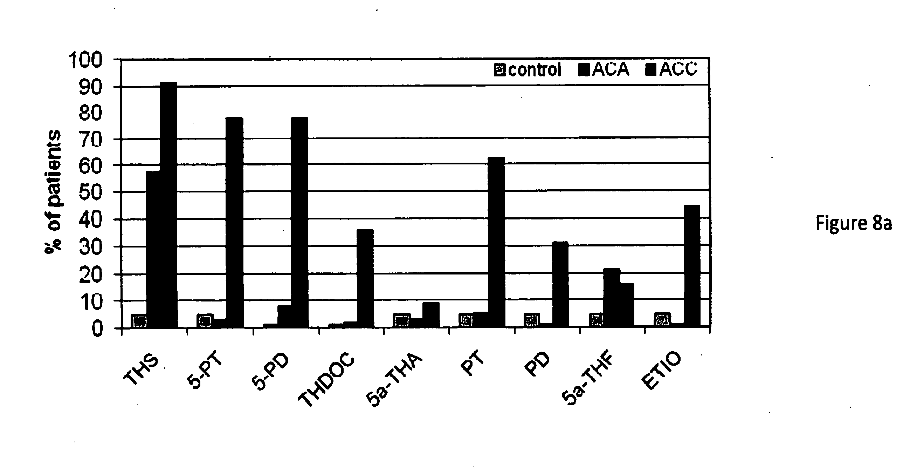

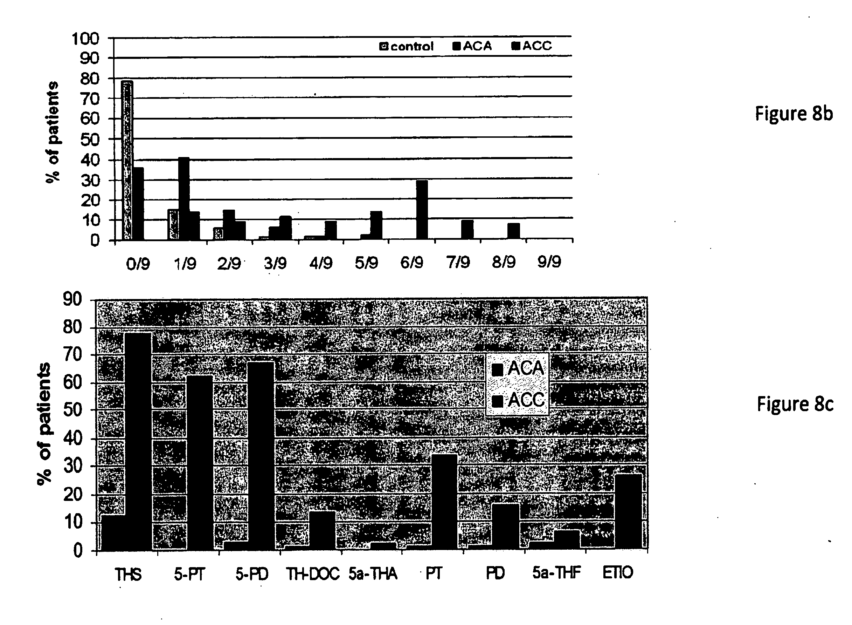

Further analysis expanded the key markers to those shown in Figure 8.

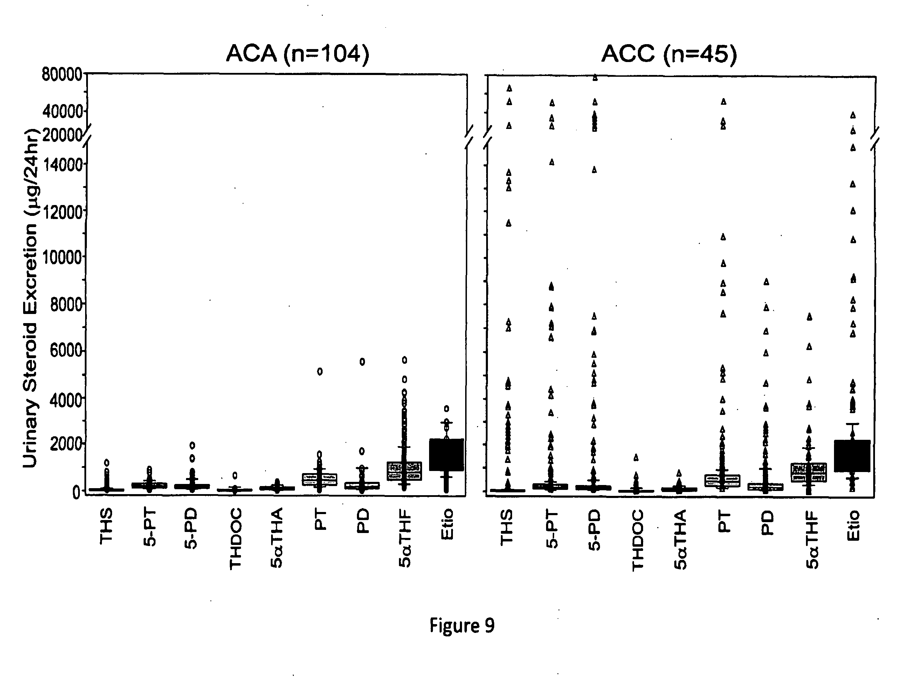

Figure 9 shows the amounts of the markers selected after MRLVQ analysis in µg/24 hours from patients. This shows some spread of concentrations. Hence, the concentration from a patient may be compared to a reference or average value, to ascertain the significance of the concentration of each marker and produce an overall score for each concentration.

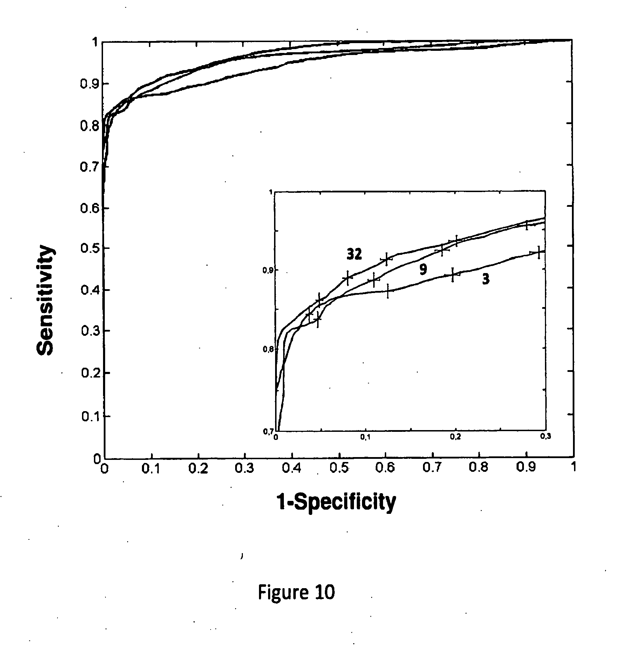

The data shows that using 6 of the 7 markers produces a highly accurate and sensitive assay, without having to compare, for example, the other steroids which could have been assigned. This is shown in Figure 10 which shows the ROC curve for 3 or 9 steroids of the invention (lower curve), compared to using 32 steroids (upper curve). Hence, selecting the markers of the invention allows a highly sensitive, selective assay to be produced. The three markers are THS, 5-PT AND 5-PD. The nine markers additionally include PD, PT, Et, TH-DOC, 5α-THA and 5α-THF.

This can be further improved by looking at 6 ß-OH-Cortisol in urine. This is especially useful for following mitotane treatment.

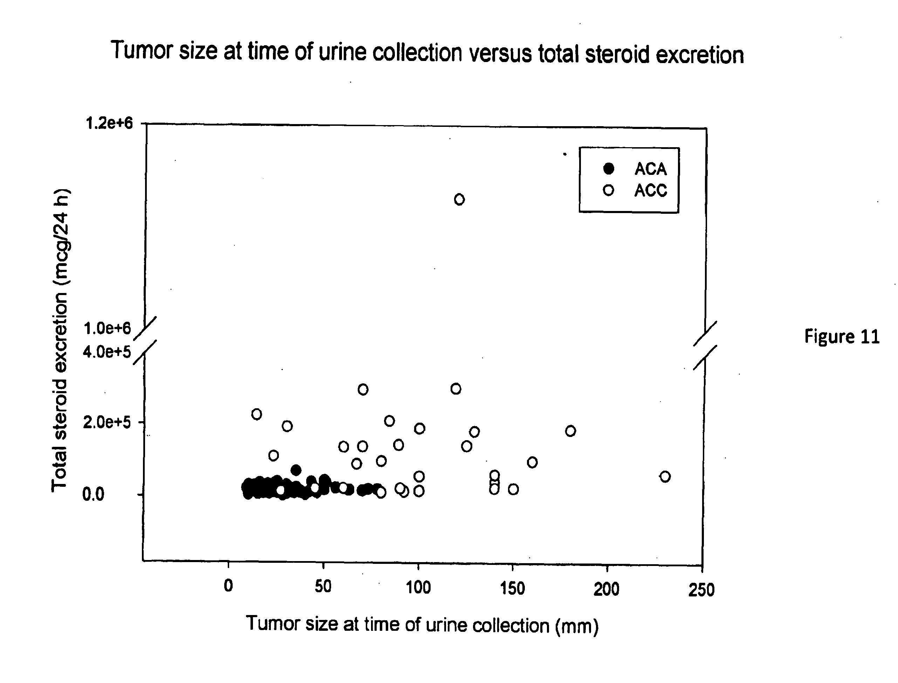

Figure 11 shows that total steroid excretion is independent of tumour size. This helps to confirm the validity of the data.

Experiments using urine samples analysed on a uPLC/tandem mass spectroscopy platform (results not shown), showed all nine preferred markers could be separated and quantified within 5 minutes using positive ion mode. This confirmed that the assay can readily be used as a high-throughput assay.

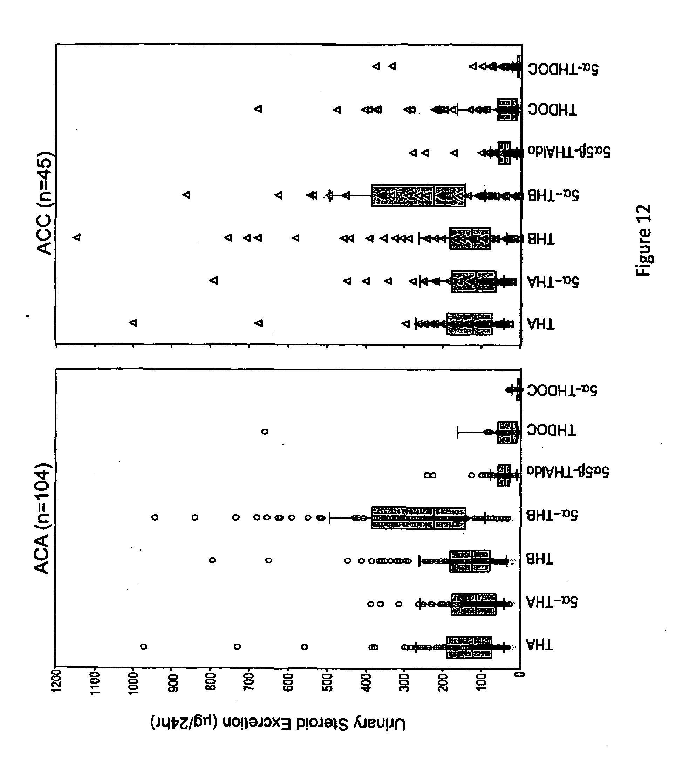

Mineralocorticoid levels are shown in Figure 12. This allows ACA to be identified. This allows the differentiation between unilateral adrenocorticoil adenoma and bilateral hyperplasia.

## Claims

1. A method of detecting or monitoring an adrenal tumour comprising measuring the concentration or amount in urine of THS (tetrahydro-11-desoxycortisol), 5-PT (5-pregnenetriol) and 5-PD (pregnenediol).

2. A method according to claim 1, additionally comprising measuring each of PD (pregnanediol), PT (pregnanetriol), Et (etiocholanolone), TH-DOC (tetrahydro-11-deoxycorticosterone), 5α-THA (5α-tetra-11-hydrocorticosterone) and 5α-THF (5α-tetrahydrocortisol).

3. A method according to any preceding claim additionally comprising measuring an (androsterone) and/or measuring the concentration or amount of 6 ß-OH-Cortisol.

4. A method according to any preceding claim comprising measuring the concentration ratio of the androsterone : etiocholanone (An : Et) in the sample.

5. A method according to any preceding claim, wherein the concentration or amount of the or each compound is compared to a predetermined value.

6. A method according to claim 5, wherein the predetermined value is a reference value associated with non-tumourous urine samples, urine samples from patients with benign tumours and/or urine samples with malignant tumours, preferably wherein the concentration or amount of the or each compound is compared to the predetermined reference value and scored to give an indication of whether the adrenal tumour is benign or malignant.

7. A method according to claim 5, wherein the urine sample is from a patient and the predetermined value is the concentration or amount of the or each compound in one or more samples of urine obtained from the patient prior to surgical removal of the adrenal tumour or prior to treatment of the adrenal tumour with one or more drugs.

8. A method for identifying or monitoring an adrenal tumour for treatment with a drug comprising comparing a concentration or amount of a compound according to any of claims 1 to 5, with the concentration or amount of the or each compound in a sample from a patient treated with that drug or one or more samples obtained from a patient not treated with the drug.

9. A method according to any preceding claim, wherein the concentration of the or each sample of the compound is determined by GC/MS (gas chromatography/mass spectroscopy) or LS/MS (liquid chromatography/mass spectroscopy).

10. A computer system having a processor, a memory and an input for receiving data obtained from a method according to any preceding claim, the computer configured to compare data obtained from the method according to any of claims 1 to 4, and having a computer program adapted to perform the method of claim 6.

11. A computer system according to claim 10, wherein the data is compared to the reference value for each compound to produce a ranking value indicative of the presence of the tumour and/or whether the tumour is benign or malignant.

12. A computer program comprising instructions which, when run on a computer with a processor, memory and input, to configure it to the computer system according to claim 10 or claim 11.

13. A kit for the measuring in urine of the concentration or amount of THS, 5-PT and 5-PD, and optionally PD, PT, TH-DOC, 5α-THA, 5α-THF and Et, comprising one or more antibodies for each compound.

14. A kit according to claim 13 additionally for the detection of An and Et in the sample and/or for the detection of 6 ß-OH-Cortisol.

15. A kit according to claims 13 and 14 which is adapted for use with GS/MS or LC/MS.

16. Adrenal tumour detection or monitoring apparatus comprising a computer system according to claims 10 or 11, and/or a computer program according to claim 12, and a kit according to claims 13 or 14.

## Patentansprüche

1. Verfahren zum Detektieren oder Beobachten eines Nebennierentumors, umfassend das Messen der Konzentration von oder der Menge an THS (Tetrahydro-11-Desoxycortisol), 5-PT (5-Pregnenetriol) und 5-PD (Pregnenediol) in Urin.

2. Verfahren nach Anspruch 1, zusätzlich umfassend das jeweilige Messen von PD (Pregnanediol), PT (Pregnanetriol), Et (Etiocholanolon), TH-DOC (Tetrahydro-11-Corticosteron), 5α-THA (5α-Tetra-11-Hydrocorticosteron), 5α-THF (5α-Tetrahydrocortisol).

3. Verfahren nach einem der vorhergehenden Ansprüche, zusätzlich umfassend das Messen von An (Androsteron) und/oder das Messen der Konzentration von oder der Menge an 6 β-OH-Cortisol.

4. Verfahren nach einem der vorhergehenden Ansprüche, umfassend das Messen des Konzentrationsverhältnisses des Androsterons : Etiocholanon (An : Et) in der Probe.

5. Verfahren nach einem der vorhergehenden Ansprüche, wobei die Konzentration oder Menge der oder jeder Verbindung mit einem vorgegebenen Wert verglichen wird.

6. Verfahren nach Anspruch 5, wobei der vorgegebene Wert ein Referenzwert ist, der nichttumorhaften Urinproben, Urinproben von Patienten mit gutartigen Tumoren und/oder Urinproben mit bösartigen Tumoren zugeordnet ist, wobei vorzugsweise die Konzentration oder Menge der oder jeder Verbindung mit dem vorgegebenen Referenzwert verglichen und bewertet wird, um einen Hinweis darauf zu geben, ob der Nebennierentumor gutartig oder bösartig ist.

7. Verfahren nach Anspruch 5, wobei die Urinprobe von einem Patienten stammt und der vorgegebene Wert die Konzentration oder Menge der oder jeder Verbindung in einer oder mehreren Urinproben des Patienten ist, die vor der operativen Entfernung des Nebennierentumors oder vor der Behandlung des Nebennierentumors mit einem oder mehreren Medikamenten von dem Patienten genommen wurden.

8. Verfahren zum Identifizieren oder Beobachten eines Nebennierentumors für eine Behandlung mit einem Medikament, umfassend das Vergleichen einer Konzentration oder Menge einer Verbindung gemäß einem der Ansprüche 1 bis 5 mit der Konzentration oder Menge der oder jeder Verbindung in einer Probe von einem Patienten, der mit diesem Medikament behandelt wurde, oder in einer oder mehreren Proben, die von einem Patienten genommen wurden, der nicht mit dem Medikament behandelt wurde.

9. Verfahren nach einem der vorhergehenden Ansprüche, wobei die Konzentration der oder jeder Probe der Verbindung mittels GC/MS (Gaschromatographie/Massenspektroskopie) oder LS/MS (Flüssigchromatographie/Massenspektroskopie) bestimmt wird.

10. Computersystem mit einem Prozessor, einem Speicher und einem Eingang für den Empfang von Daten, die mit einem Verfahren gemäß einem der vorstehenden Ansprüche ermittelt wurden, wobei der Computer konfiguriert ist für den Vergleich der von einem Verfahren gemäß einem der Ansprüche 1 bis 4 empfangenen Daten und ein Computerprogramm enthält, das für die Durchführung des Verfahrens gemäß Anspruch 6 ausgebildet ist.

11. Computersystem nach Anspruch 10, wobei die Daten mit dem Referenzwert für jede Verbindung verglichen werden, um einen Rangwert zu erzeugen, der auf das Vorhandensein des Tumors und/oder darauf hinweist, ob der Tumor gutartig oder bösartig ist.

12. Computerprogramm, das Anweisungen enthält zum Konfigurieren desselben für das Computersystem gemäß Anspruch 10 oder 11, wenn das Computerprogramm auf einem Computer mit einem Prozessor, einem Speicher und einer Eingabe läuft.

13. Kit zum Messen der Konzentration von oder der Menge an THS, 5-PT und 5-PD und optional PD, PT, TH-DOC, 5α-THA, 5α-THF und Et in Urin, enthaltend einen oder mehrere Antikörper für jede Verbindung.

14. Kit nach Anspruch 13, zusätzlich zum Detektieren von An und Et in der Probe und/oder zum Detektieren von 6 β-OH-Cortisol.

15. Kit nach Anspruch 13 und 14, das geeignet ist für die Verwendung mit GS/MS oder LC/MS.

16. Vorrichtung zum Detektieren oder Beobachten eines Nebennierentumors, umfassend ein Computersystem gemäß den Ansprüchen 10 oder 11 und/oder ein Computerprogramm gemäß Anspruch 12 und ein Kit gemäß den Ansprüchen 13 oder 14.

## Revendications

1. Procédé permettant de détecter ou de surveiller une tumeur surrénale comprenant une étape consistant à mesurer la concentration ou la quantité dans l'urine de THS (tetrahydro 11 desoxy cortisol), de 5-PT (5-pregnenetriol) et de 5-PD (pregnenediol).

2. Procédé conforme à la revendication 1, comprenant en outre une étape consistant à mesurer chacun des composés suivants PD (pregnenediol), PT (pregnanetriol), Et (ethiocholanolone), TH-DOC (tetrahydro 11 corticosterone), 5α-THA (5α-tetra 11 hydrocorticosterone) et 5α-THF (5α-tetrahydrocortisol).

3. Procédé conforme à l'une quelconque des revendications précédentes, comprenant en outre une étape consistant à mesurer (l'Androstérone) et/ou à mesurer la concentration ou la quantité de 6 β-OH-Cortisol.

4. Procédé conforme à l'une quelconque des revendications précédentes, comprenant une étape consistant à mesurer le rapport de concentration androsterone : etiocholanone (An : Et) dans l'échantillon.

5. Procédé conforme à l'une quelconque des revendications précédentes, selon lequel la concentration ou la quantité du ou de chacun des composés est comparée à une valeur prédéfinie.

6. Procédé conforme à la revendication 5, selon lequel la valeur prédéfinie est une valeur de référence associée à des échantillons d'urine non tumorale, des échantillons d'urine de patients ayant des tumeurs bénignes et/ou d'échantillons d'urine avec des tumeurs malignes, de préférence, la concentration ou la quantité du ou de chacun des composés étant comparée à la valeur de référence prédéfinie et marquée d'un score pour donner une indication permettant de déterminer si la tumeur surrénale est bénigne ou maline.

7. Procédé conforme à la revendication 5, selon lequel l'échantillon d'urine est un échantillon d'un patient et la valeur prédéfinie est la concentration ou la quantité du ou de chacun des composés dans au moins un échantillon d'urine obtenu chez un patient avant l'ablation chirurgicale de la tumeur surrénale ou avant le traitement de la tumeur surrénale avec un ou plusieurs médicaments.

8. Procédé permettant d'identifier ou de surveiller une tumeur surrénale de façon à permettre son traitement avec un médicament comprenant une étape consistant à comparer la concentration ou la quantité d'un composé conforme à l'une quelconque des revendications 1 à 5, avec la concentration ou la quantité du ou de chacun des composés dans un échantillon d'un patient traité avec ce médicament ou un ou plusieurs échantillons obtenus chez un patient non traité avec le médicament.

9. Procédé conforme à l'une quelconque des revendications précédentes, selon lequel la concentration du ou de chacun des échantillons du composé est déterminé par GC/MS (chromatographie en phase gazeuse /spectroscopie de masse) ou par LS/MS (chromatographie en phase liquide / spectroscopie de masse).

10. Système d'ordinateur comprenant un processeur, une mémoire et une entrée permettant de recevoir des données obtenues lors de la mise en oeuvre du procédé conforme à l'une quelconque des revendications précédentes, cet ordinateur étant conformé pour comparer des données obtenues par la mise en oeuvre du procédé conforme à l'une quelconque des revendications 1 à 4, et ayant un programme d'ordinateur adapté pour mettre en oeuvre le procédé conforme à la revendication 6.

11. Système d'ordinateur conforme à la revendication 10, selon lequel les données sont comparées à la valeur de référence pour chacun des composés de façon à obtenir une valeur de niveau indiquant la présence de la tumeur et/ou si cette tumeur est bénigne ou maligne.

12. Programme d'ordinateur comprenant des instructions qui, lorsqu'elles sont exécutées sur un ordinateur avec un processeur une mémoire et une entrée permettent de le conformer selon le système d'ordinateur conforme à la revendication 10 ou à la revendication 11.

13. Kit permettant de mesurer dans l'urine la concentration ou la quantité de THS, de 5-PT de 5-PD et le cas échéant de PD, de PT, de TH-DOC, de 5α-THA, de 5α-THF et d'Et comprenant un ou plusieurs anti-corps de chacun des composés.

14. Kit conforme à la revendication 13, pour en outre la détection de An et Et dans l'échantillon et/ou pour la détection de 6 β-OH Cortisol.

15. Kit conforme aux revendications 13 et 14 qui est adapté pour être utilisé par GS/MS ou par LC/MS.

16. Appareil de détection ou de surveillance de tumeur surrénale comprenant un système d'ordinateur conforme aux revendications 10 ou 11 et/ou un programme d'ordinateur conforme à la revendication 12 et un kit conforme aux revendications 13 ou 14.
